# EUROPÄISCHE PATENTSCHRIFT

(11) **EP 4 444 230 B1**
(45) Veröffentlichungstag und Bekanntmachung des Hinweises auf die Patenterteilung: **12.03.2025**
(21) Anmeldenummer: 22830144.6
(22) Anmeldetag: 01.12.2022
(51) Int. Cl.: A61F 2/68, A61F 2/70

(54) **VERFAHREN ZUM ERZEUGEN EINES AKUSTISCHEN FEEDBACKS UND ORTHOPÄDIETECHNISCHE EINRICHTUNG**
METHOD FOR GENERATING ACOUSTIC FEEDBACK, AND ORTHOPAEDIC DEVICE
PROCÉDÉ POUR GÉNÉRER UNE RÉTROACTION ACOUSTIQUE ET DISPOSITIF ORTHOPÉDIQUE

(30) Priorität: 07.12.2021 DE 102021132188
(43) Veröffentlichungstag der Anmeldung: 16.10.2024
(73) Patentinhaber: Otto Bock Healthcare Products GmbH, 1110 Wien (AT)
(72) Erfinder: INSCHLAG, Josef, 1110 Wien (AT); EDER, Marcus, 1110 Wien (AT)
(74) Vertreter: Gramm, Lins & Partner Patent- und Rechtsanwälte PartGmbB
(86) Internationale Anmeldenummer: PCT/EP2022/084045
(87) Internationale Veröffentlichungsnummer: WO 2023/104629

(56) Entgegenhaltungen:
- EP-A1- 2 495 853
- US-A1- 2020 306 060

## Beschreibung

Die Erfindung betrifft ein Verfahren zum Erzeugen eines akustischen Feedbacks einer orthopädietechnischen Einrichtung mit zumindest einem Motor, der eine Steuerungseinrichtung, einen Stator und einen um eine Drehachse verdrehbaren Rotor aufweist und mit einer durch den Motor verstellbaren Komponente der orthopädietechnischen Einrichtung gekoppelt ist sowie eine solche orthopädietechnische Einrichtung.

Akustische Feedbacks werden an orthopädietechnischen Einrichtungen verwendet, um dem Anwender bestimmte Informationen mitzuteilen, wenn beispielsweise eine bestimmte Bewegung ausgeführt wird, eine bestimmte Griffkraft erreicht wird, ein Objekt berührt wird oder ein Bewegungs- oder Griffmuster abgeschlossen wurde. Auf diese Weise erleichtern akustische Feedbacks dem Anwender den Umgang mit der orthopädietechnischen Einrichtung und geben ihm darin Sicherheit.

Die EP 3 753 536 A1 offenbart ein Rückmeldesystem für eine Prothese mit einem Handschuh, einem an dem Handschuh angeordneten Sensor, der ein Messsignal erzeugt, und mindestens zwei einander ergänzende Rückmeldungseinrichtungen, die das Messsignal in ein Feedback-Signal umwandeln. Die Rückmeldungseinrichtungen können als Leuchtelement, als Vibrationsmotor, als Druckaktuator oder als Lautsprecher ausgebildet sein.

Die US 2020/0306060 A1 betrifft ein System, bestehend aus mindestens einer orthopädischen Komponente, mit mindestens einem Energiespeicher und mehreren elektrischen und/oder elektronischen Einrichtungen sowie mindestens einer Bedienungs- und/oder Rückmeldeeinrichtung, die den elektrischen und/oder elektronischen Einrichtungen zugeordnet und mit diesen gekoppelt ist.

Die EP 2 495 853 A1 betrifft einen Magneto-elektrischen Motor, der aus einem Motorkörper mit einem Stator mit einem ferromagnetischen Kern in Ringform mit aufgewickelten Wicklungen und einem Rotor mit gleichmäßig um ferromagnetische Scheiben verteilten Permanentmagneten sowie einem externen Gehäuse mit Induktoren und Kondensatoren besteht, die zusammen mit den Statorwicklungen einen in den Motor integrierten elektrischen Schwingkreis bilden. Aufgrund der Wechselwirkung zwischen dem permanent verfügbaren magnetischen Moment der auf den Rotorscheiben sitzenden Magnete und der im Luftspalt des Motors induzierten magnetischen Flussdichte aufgrund der selbst und permanent erzeugten Ströme im elektrischen Schwingkreis, die den Statorwicklungen zugeführt werden, ist der Motor in der Lage, in einem Selbstantriebsmodus auf Basis der intern entwickelten Energie zu arbeiten. Ein Motordrehmoment wird nach dem Prinzip der elektromechanischen Energieumwandlung erzeugt. Aufgrund der Selbstkommutierungsfähigkeit des Motors ist das Drehmoment unidirektional und darüber hinaus wird durch die Winkelverschiebung des Rotors eine kontinuierliche Drehbewegung der Motorwelle sichergestellt.

Bisweilen werden akustische Feedbacks an orthopädietechnischen Einrichtungen durch separate Lautsprecher, Vibrationsmotoren oder Druckaktuatoren ausgeführt. Dadurch entstehen Mehrkosten für die Bereitstellung der Bauteile, die zudem einen Teil des begrenzten Bauraums in Anspruch nehmen.

Aufgabe der vorliegenden Erfindung ist es, ein Verfahren sowie eine Vorrichtung zum Erzeugen eines akustischen Feedbacks bereitzustellen, die kostengünstig und platzsparend sind.

Diese Aufgabe wird erfindungsgemäß durch ein Verfahren mit den Merkmalen des Hauptanspruchs und eine orthopädietechnische Einrichtung mit den Merkmalen des nebengeordneten Anspruchs gelöst. Vorteilhafte Ausgestaltungen und Weiterbildungen der Erfindung sind in den Unteransprüchen, der Beschreibung sowie den Figuren offenbart.

Das Verfahren zum Erzeugen eines akustischen Feedbacks einer orthopädietechnischen Einrichtung mit zumindest einem Elektromotor, der einen Stator und einen um eine Drehachse verdrehbaren Rotor aufweist und mit einer durch den Elektromotor verstellbaren Komponente der orthopädietechnischen Einrichtung gekoppelt ist, sieht vor, dass der Motor durch wiederholte Umpolung zumindest einer Motorspannung oszillierend betrieben wird, ohne dass der Rotor eine vollständige Drehung um die Drehachse ausführt. Die Bewegung des Rotors verursacht ein akustisches Geräusch, das als Signal für den Anwender einer orthopädietechnischen Einrichtung dienen kann. Da es sich aufgrund der wiederholten, insbesondere periodischen Umpolung um eine oszillierende Bewegung handelt, führt der Rotor nur kleine Schwingungen oder Drehbewegungen um seine Drehachse aus, sodass eine an den Elektromotor gekoppelte Komponente der orthopädietechnischen Einrichtung nicht oder nur minimal bewegt wird.

Eine Weiterbildung sieht vor, dass als Motorspannung eine Rechteckspannung, Dreiecksspannung, Sägezahnspannung, Sinuskurvenspannung oder eine Mischform daraus verwendet wird. Derartige Wellenformen dienen als Muster für die Umpolung, das bei der Erzeugung eines akustischen Geräusches mehrfach wiederholt werden kann. Die jeweilige Wellenform beeinflusst dabei maßgeblich die Klangfarbe des erzeugten akustischen Feedbacks.

Bevorzugt wird die Umpolung mit einer Frequenz zwischen 20 Hz und 6000 Hz betrieben, kann aber auch darüber hinausgehen und bis zur menschlichen Hörschwelle reichen. Eine höhere Umpolfrequenz erzeugt ein akustisches Feedback mit einer höheren Frequenz. Bei einer periodischen Umpolung hängt die Tonhöhe des akustischen Feedbacks von der Frequenz der Umpolung ab.

Vorteilhafterweise wird ein Elektromotor mit unterschiedlichen Umpolfrequenzen in zeitlicher Abfolge und/oder werden mehrere Elektromotoren mit unterschiedlichen Umpolfrequenzen gleichzeitig oder nacheinander betrieben. Auf diese Weise ist es einerseits möglich, eine Abfolge von mehreren Tönen und/oder Geräuschen zu erzeugen, andererseits können durch die gleichzeitige Verwendung mehrerer Elektromotoren polyphone Klänge erzeugt werden.

Bevorzugt wird die Amplitude der Motorspannung während der Umpolung verändert. Bei einer Änderung der Amplitude der Motorspannung während der Umpolung wird die Lautstärke des akustischen Feedbacks beeinflusst. So können Töne und Geräusche mit unterschiedlicher Lautstärke oder auch solche mit steigender oder sinkender Lautstärke erzeugt werden.

Eine Weiterbildung sieht vor, dass der Motor als Kommutatormotor ausgebildet ist und die Motorspannung des Elektromotors zum Erzeugen eines akustisch wahrnehmbaren Signals wiederholt, insbesondere periodisch umgepolt wird. Kommutatormotoren sind günstig herzustellen und erfordern keine komplexe Steuerung. Aufgrund der Reibung der Schleifkontakte am Kommutator erzeugen Kommutatormotoren für gewöhnlich lautere Geräusche als bürstenlose Gleichstrommotoren.

Eine Variante sieht vor, dass der Motor als bürstenloser Gleichstrommotor ausgebildet ist und die Phasenlage des Drehfeldes des Elektromotors zum Erzeugen eines akustisch wahrnehmbaren Signals wiederholt, insbesondere periodisch geändert wird. Bürstenlose Gleichstrommotoren weisen eine geringe Wärmeentwicklung auf und haben daher höhere Standzeiten. Darüber hinaus zeichnen sich bürstenlose Gleichstrommotoren durch eine hohe Effizienz aus und sind daher bei gleicher Leistung in der Regel kleiner als Kommutatormotoren.

Vorteilhafterweise wird der Rotor zur Erzeugung eines akustisch wahrnehmbaren Signals um einen Drehwinkel von nicht mehr als 120°, insbesondere nicht mehr als 90°, oszillierend bewegt. In gewöhnlichen Antriebssträngen von Komponenten einer orthopädietechnischen Einrichtung führen derartige Drehwinkel nur zu einer minimalen oder zu gar keiner Bewegung der jeweiligen angetriebenen Komponente, sodass bei der Erzeugung eines akustisch wahrnehmbaren Signals keine signifikante ungewollte Bewegung ausgeführt wird. Die Antriebsstrangkomponenten weisen beispielsweise ein Getriebe oder ein mechanisches Spiel auf, sodass bei kleinen Drehwinkeln in der Regel nur die Antriebsstrangkomponenten, nicht jedoch die angetriebene Komponente der orthopädietechnischen Einrichtung, bewegt werden. Der Motor bewegt sich, aber nur sehr minimal. Je höher die Frequenz der Umpolung, desto kleiner die Bewegung des Rotors. Aufgrund der Massenträgheit kann der Motor den Richtungsänderungen bei höheren Frequenzen immer weniger folgen. Eine minimale Bewegung des Rotors ist für die Erzeugung eines hörbaren Signals aber notwendig. Die Amplitude der Rotorschwingung ist vergleichbar mit der eines Piezolautsprechers. Je niedriger die Frequenz, desto lauter ist das Signal.

Die orthopädietechnische Einrichtung mit zumindest einem Elektromotor, der einen Stator und einen um eine Drehachse verdrehbaren Rotor aufweist und mit einer durch den Elektromotor verstellbaren Komponente der orthopädietechnischen Einrichtung gekoppelt und mit einer Steuerungseinrichtung verbunden ist, sieht vor, dass die Steuerungseinrichtung eingerichtet ist, um zumindest eine Motorspannung des Elektromotors zum Erzeugen eines akustisch wahrnehmbaren Signals wiederholt umzupolen, ohne dass der Rotor eine vollständige Drehung um die Drehachse ausführt. Dadurch, dass ein Elektromotor einer verstellbaren Komponente der orthopädietechnischen Einrichtung zum Erzeugen eines akustisch wahrnehmbaren Signals verwendet wird, sind keine weiteren Lautsprecher, andere Einrichtungen zum Erzeugen eines akustischen Feedbacks oder separate Elektromotoren zum Erzeugen des akustischen Feedbacks notwendig. Dadurch können zum einen die Kosten der orthopädietechnischen Einrichtung gesenkt werden. Zum anderen fällt der Bauraum für die separaten Komponenten, die sonst zur Erzeugung eines akustischen Feedbacks nötig sind, weg und steht somit für andere Komponenten oder zur Verkleinerung der orthopädietechnischen Einrichtung zur Verfügung. Da der Rotor des Elektromotors bei der Erzeugung des akustischen Feedbacks nur eine unvollständige Drehung um die Drehachse ausführt, wird die durch den Elektromotor verstellbare Komponente der orthopädietechnischen Einrichtung nur minimal oder gar nicht bewegt, sodass keine Nachteile hinsichtlich der Bedienung durch den Anwender entstehen.

Vorteilhafterweise ist die Steuerungseinrichtung eingerichtet, eine Umpolfrequenz zwischen 20 Hz und 6000 Hz oder mehr zu erzeugen. Mit Umpolfrequenzen aus diesem Bereich lassen sich ausreichende Lautstärken für ein akustisches Feedback erzielen. Die Tonhöhen liegen dabei im Frequenzbereich des menschlichen Gehörs. Zugleich sind die benötigen Spannungsamplituden in diesem Umpolfrequenzbereich relativ gering.

Bevorzugt liegen bei unterschiedlichen Umpolfrequenzen unterschiedliche Spannungsamplituden an. Durch die Verwendung verschiedener frequenzabhängiger Spannungsamplituden ist es möglich, die maximale Drehung auch bei verschiedenen Frequenzen gleichbleibend zu gestalten.

Eine Weiterbildung sieht vor, dass der Elektromotor als bürstenloser Gleichstrommotor oder Kommutatormotor ausgebildet ist. Kommutatormotoren sind aufgrund ihres einfachen Aufbaus günstig herzustellen, während bürstenlose Gleichstrommotoren sich durch eine hohe Effizienz auszeichnen.

Nachfolgend wird ein Ausführungsbeispiel der Erfindung anhand der Figuren 1 bis 3 näher erläutert. Gleiche Bezugszeichen bezeichnen gleiche Bauteile. Es zeigen:
Figur 1 - eine schematische Darstellung einer orthopädietechnischen Einrichtung;
Figur 2a - einen Kommutatormotor;
Figur 2b - ein Diagramm der Motorspannung über der Zeit;
Figur 3a - einen bürstenlosen Gleichstrommotor;
Figur 3b - ein Diagramm des Motorwinkels sowie der Polspannungen über der Zeit; sowie
Figur 4 - eine schematische Darstellung einer Prothese der oberen Extremität.

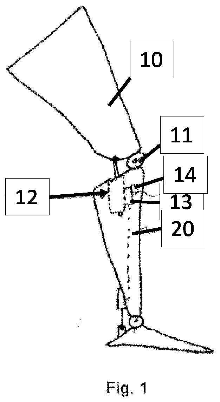

Figur 1 zeigt in einer schematischen Darstellung eine orthopädietechnische Einrichtung in Gestalt einer Prothese einer unteren Extremität mit einem Oberteil 10 und einem Unterteil 20, die gelenkig aneinander um eine Schwenkachse 11 gelagert sind. In dem dargestellten Ausführungsbeispiel weist das Oberteil 10 einen Prothesenschaft auf, der zur Aufnahme eines Oberschenkelstumpfes dient. Andere orthopädietechnische Einrichtungen wie Prothesen der oberen Extremität sowie Orthesen der oberen und unteren Extremität sind weitere Beispiele für eine orthopädietechnische Einrichtung. Zwischen dem Oberteil 10 und dem Unterteil 20 ist ein Aktuator 12 angeordnet. Der Aktuator 12 ist im dargestellten Ausführungsbeispiel als ein linear wirkender Aktuator 12 ausgebildet und weist einen Antrieb 13 in Gestalt eines Elektromotors auf, um eine Verstellung der Position des Oberteils 10 relativ zu dem Unterteil 20 zu bewirken. Der Aktuator 12 kann beispielsweise als ein hydraulisch wirkender Aktuator ausgebildet sein und eine Pumpe aufweisen, die durch den Antrieb 13 angetrieben wird. Über die Pumpe wird ein Hydraulikfluid in eine Pumpenkammer oder in eine von einem Kolben verschlossene Kammer gepumpt. Der Kolben ist mit einer Kolbenstange gekoppelt, die an dem Oberteil 10 oder Unterteil 20 gelagert ist. Durch entsprechendes Verpumpen des Hydraulikfluids in die Pumpenkammer wird die Kolbenstange in die eine oder andere Richtung bewegt und das Oberteil 10 relativ zu dem Unterteil 20 verschwenkt. Dadurch ist es möglich, eine Extensionsbewegung oder eine Flexionsbewegung der orthopädietechnischen Einrichtung auszuführen, zu bremsen oder zu unterstützten. Alternativ zu einer hydraulischen Ausgestaltung des Aktuators 12 kann dieser auch mechanisch ausgebildet sein, sodass über den Antrieb 13 beispielsweise eine Spindel angetrieben wird. Die Spindel kann dann in ein Aktuatorgehäuse einfahren und ausfahren, um eine Extension oder Flexion der orthopädietechnischen Einrichtung zu bewirken. An dem Antrieb 13 ist eine Steuerungseinrichtung 14 angeordnet, die eine wiederholte Umpolung einer Motorspannung des Antriebs 13 erlaubt, sodass dieser eine oszillierende Bewegung ausführt, mit der ein akustisches Feedback erzeugt wird.

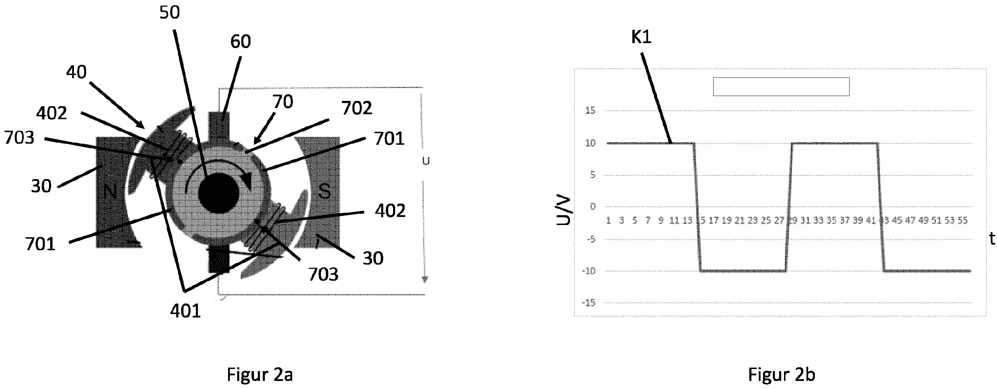

In der Figur 2a ist ein Kommutatormotor gezeigt. Der Kommutatormotor weist einen Stator 30 auf, der als Permanentmagnet mit einem magnetischen Nordpol N und einem magnetischen Südpol S ausgeführt ist. Zwischen den beiden magnetischen Polen ist der Rotor 40 angeordnet. Der Rotor 40 ist um eine Drehachse 50 schwenkbar und ist in dem gezeigten Ausführungsbeispiel als Doppel-T-Rotor mit zwei T-förmigen Kopfelementen 401,

die entlang einer Achse, die quer zur Drehachse 50 steht, symmetrisch angeordnet sind. Konzentrisch zu der Drehachse 50 ist ein zylindrischer Kommutator 70 an dem Rotor 40 angeordnet, der fest mit diesem verbunden ist. An zwei gegenüberliegenden Seiten des Kommutators 70 sind
Schleifkontakte 60 angeordnet. An der Mantelfläche des Kommutators 70 sind zwei leitenden Zonen 701 angeordnet. Die beiden leitenden Zonen 701 sind durch eine nichtleitende Zone 702 voneinander räumlich getrennt. Die nicht leitende Zone 702 erstreckt sich an zwei gegenüberliegenden Bereichen bis an die Mantelfläche des Kommutators 70 und teilt somit die leitende Zone 701 in zwei Bereiche gleicher Größe auf. Die leitenden Zonen 701 sind über jeweils einen Anschluss 703 mit der Rotorwicklungen 402, die an den zylindrischen Abschnitten der beiden T-förmigen Kopfelemente 401 angeordnet sind, verbunden. In der gezeigten Lage berühren die Schleifkontakte 60 die leitenden Zonen 701. Da an den Schleifkontakten 60 eine Motorspannung U anliegt, wird am Rotor über die Rotorwicklung 402 ein elektromagnetisches Feld erzeugt. Aufgrund der Anziehung und Abstoßung von dem Permanentmagneten des Stators 30 beginnt sich der Rotor 40 zu drehen. Bei der Rotation dient der Kommutator 70 als mechanischer Schalter zum Umpolen des elektromagnetischen Feldes am Rotor 40, sodass eine durchgängige Rotation ermöglicht wird. Durch eine Steuerungseinrichtung 14 lässt sich die Motorspannung U, welche an den Schleifkontakten 60 anliegt, umpolen. Eine derartige Umpolung hat zur Folge, dass der Rotor 40 einen Elektromagneten mit der gegensätzlichen Polung ausbildet. Dadurch wird eine Rotation des Rotors 40 in der entgegengesetzten Richtung veranlasst. Erfolgt die Umpolung durch die Steuerungsrichtung 14 wiederholt, insbesondere periodisch, führt der Rotor 40 eine oszillierende Bewegung aus, mit der ein akustisches Feedback erzeugt werden kann.

Figur 2b zeigt ein Diagramm der Motorspannung U an einem Kommutatormotor über der Zeit. Die Kurve K1 zeigt einen rechteckförmigen Verlauf der Motorspannung U über der Zeit t. Zu Beginn beträgt die Motorspannung 10 Volt, nach 14 Zeiteinheiten wechselt diese sprunghaft zu -10 Volt, nach weiteren 14 Zeiteinheiten springt die Motorspannung erneut auf 10 Volt. Die Umpolung erfolgt periodisch. Aperiodische Umpolungen sind ebenfalls möglich. Als Wellenform für die Umpolung wird hier eine Rechteckform verwendet. Es können auch dreieckförmige, sägezahnförmige oder sinusförmige Wellenformen verwendet werden oder eine Mischform aus diesen. Die Frequenz der dargestellten Wellenform beträgt etwa 36 Hz bei einer Zeiteinheit von t in Millisekunden, es sind auch andere Frequenzen, insbesondere mehreren überlagerte Frequenzen denkbar, die im Hörbereich des menschlichen Ohres liegen.

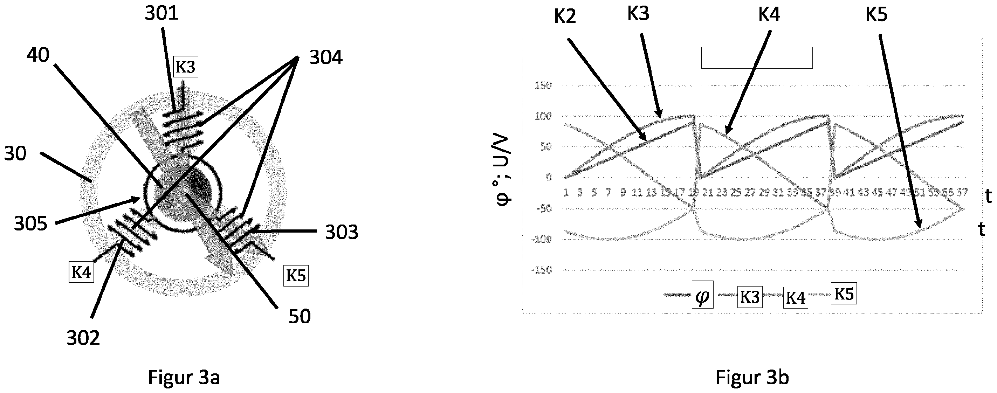

In der Figur 3a ist ein bürstenloser Gleichstrommotor gezeigt. Bei dem bürstenlosen Gleichstrommotor ist der Rotor 40 als zylindrischer Permanentmagnet ausgeführt, der sich konzentrisch innerhalb des Stators 30 befindet, einen Nordpol N sowie einen Südpol S aufweist und um die Drehachse 50 schwenkbar ist. Der Stator 30 ist als zylindrische Hülse ausgeführt, die drei nach innen vorstehende, zylindrische, ferromagnetische Kerne 301-303 aufweist, an denen jeweils eine Wicklung 304 angeordnet ist. Die Kerne 301-303 stehen in einem Winkel von 120° zueinander angeordnet. Die Wicklungen 304 der drei Kerne 301-303 stehen über ein nicht näher detailliertes Schaltungssystem miteinander in Verbindung. Über das Schaltungssystem werden an den verschiedenen Wicklungen 304 unabhängige Motorspannungen U, V, W angelegt, sodass an den drei Kernen 301 - 303 unabhängige Elektromagneten entstehen. Durch eine geeignete zeitliche Umpolung der Motorspannungen kann der Rotor 40zur Drehung um die Drehachse 50 veranlasst werden. Beispielsweise U, V und W phasenverschobene, sinusförmige Wechselspannungen sein. Wird die Wechselspannung am zweiten Kern 302 um 120° und die am dritten Kern 303 um 240° im Vergleich zum ersten Kern 301 verschoben, wird eine konstante Rotation des Rotors 40 um die Drehachse 50 synchron zur Wechselspannung hervorgerufen. An dem Stator 30 können auch nur zwei oder mehr als drei Kerne angeordnet sein.

Figur 3b zeigt ein Diagramm des Rotorwinkels ϕ sowie der Motorspannungen U, V, W an einem bürstenlosen Gleichstrommotor über der Zeit. Die Kurve K2 zeigt den Verlauf des Rotorwinkels ϕ über der Zeit. Der Rotorwinkel ϕ startet bei 0° nimmt zunächst linear bis zu einem Wert von 90° zu und sinkt dann sprunghaft wieder auf den Wert Ausgangswert von 0°. Danach startet dieser Vorgang erneut. Die Kurven K3, K4 und K5 zeigen die Verläufe der Motorspannungen U, V, W über der Zeit. Die Motorspannungen U, V, W starten bei unterschiedlichen Anfangswerten und zeigen zunächst den Verlauf von drei jeweils um 120° phasenverschobenen Sinuskurven mit einer Amplitude von 100 V. Die erste Motorspannung U steigt beispielsweise in dem Zeitfenster, in dem der Rotorwinkel bis auf 90° ansteigt, sinusförmig bis zu dem Maximalwert von 100 V an. Um den Rotorwinkel ϕ sprunghaft auf den Ausgangswert von 0° zu führen, werden die Motorspannungen U, V, W ebenfalls auf ihren jeweiligen Ausgangswert umgepolt. Dies ist nur möglich, da der Rotor 40 nur eine kleine Drehung ausgeführt hat. Wenn der Rotor 40 eine Drehung von mehr als einer Drehung durchgeführt hat, kann er nicht durch eine sprunghafte Umpolung diese Drehung rückgängig machen, sondern nur in seine relative Ausgangslage versetzt werden, bei der er jedoch im Vergleich zur Ausgangslage eine Drehung von 360° oder einem ganzzahligen Vielfachen davon ausgeführt hat.

Unabhängig von der Bauart des Elektromotors 13 ist es durch die wiederholte, insbesondere periodische Umpolung zumindest einer Motorspannung möglich, den Elektromotor 13 oszillierend zu betreiben, ohne dass der Rotor 40 eine vollständige Drehung um die Drehachse 50 ausführt. Vorteilhafterweise wird die Umpolung so durchgeführt, dass keine der Komponenten der orthopädietechnischen Einrichtung bewegt wird. Aufgrund der Fertigungstoleranzen, des notwendigen Spieles sowie der notwendigerweise zwischen dem Elektromotor 13 und den anzutreffenden Komponenten angeordneten Getriebeeinrichtungen oder Übersetzungseinrichtungen kann der Rotor 40 vergleichsweise große Drehwinkel von unter 120° ausführen, ohne dass eine Relativverlagerung der Komponenten stattfindet. Insbesondere wird der Drehwinkel von weniger als 90° eingestellt, wobei sich der Rotor 40 je nach Frequenz und Mechanik nahezu gar nicht bewegt, sondern nur ein Drehmoment erzeugt, dass periodisch das Vorzeichen wechselt. Die Bewegung oder das Wechseln des Drehmomentes reicht aus, um ein akustisches Feedback zu erzeugen, dass der Nutzer der orthopädietechnischen Einrichtung wahrnehmen kann. Das akustische wahrnehmbare Signal liegt vorteilhafterweise in einem Frequenzbereich zwischen 20 Hz und 6000 Hz.

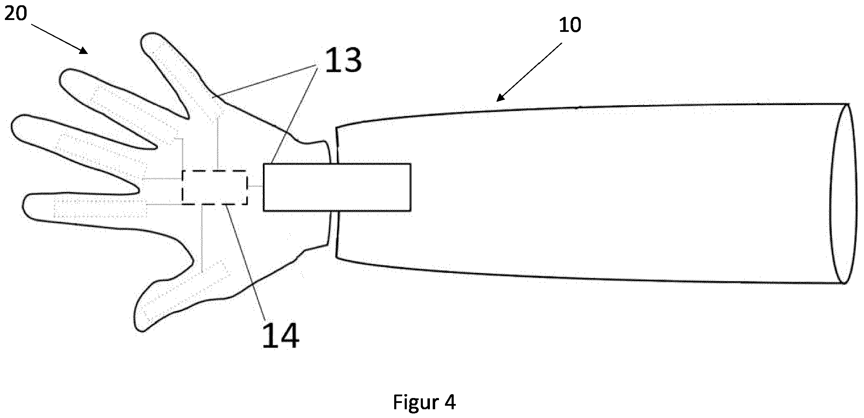

In der Figur 4 ist eine weitere Ausgestaltungsform der orthopädietechnischen Einrichtung in Gestalt einer Prothese der oberen Extremität dargestellt. Die orthopädietechnische Einrichtung weist einen Prothesenschaft als Oberteil 10 zur Aufnahme eines Unterarmstumpfes und eine distal daran angeordnete Prothesenhand als Unterteil 20 auf. Innerhalb der Prothesenhand 20 sind mehrere Elektromotoren 13 angeordnet, ein Elektromotor 13 dient zur Bewegung der Prothesenhand 20 relativ zu dem Prothesenschaft 10, während innerhalb der Prothesenhand 20 mehrere Elektromotoren 13 angeordnet sind, die die Finger bzw. den Daumen relativ zu einem Grundkörper der Prothesenhand 20 oder relativ zu einem Chassis verlagern. Alle Elektromotoren 13 sind mit einer Steuerungseinrichtung 14 gekoppelt, über die es möglich ist, die Spannung jeweiligen Motoren 13 wiederholt, umzupolen, sodass der jeweilige Elektromotor 13 oszilliert, ohne dass der Rotor des Elektromotors 13 eine vollständige Drehung um seine Drehachse ausführt.

### Bezugszeichenliste

- 10: Oberteil
- 11: Schwenkachse
- 12: Aktuator
- 13: Antrieb
- 14: Steuerungseinrichtung
- 20: Unterteil
- 30: Stator
- 301: Erster Kern
- 302: Zweiter Kern
- 303: Dritter Kern
- 304: Wicklung
- 305: Schaltungssystem
- 40: Rotor
- 401: Kopfelemente
- 402: Rotorwicklung
- 50: Drehachse
- 60: Schleifkontakt
- 70: Kommutator
- 701: Leitende Zone
- 702: Nichtleitende Zone
- K1: Motorspannung über der Zeit
- K2: Rotorwinkel über der Zeit
- K3: Erste Motorspannung über der Zeit
- K4: Zweite Motorspannung über der Zeit
- K5: Dritte Motorspannung über der Zeit
- N: Magnetischer Nordpol
- S: Magnetischer Südpol
- U: Motorspannung
- V: Zweite Motorspannung
- W: Dritte Motorspannung
- ϕ: Winkel des Rotors

## Patentansprüche

1. Orthopädietechnische Einrichtung mit zumindest einem Elektromotor (13), der einen Stator und einen um eine Drehachse (50) verdrehbaren Rotor (40) aufweist und mit einer durch den Elektromotor (13) verstellbaren Komponente (10, 20) der orthopädietechnischen Einrichtung gekoppelt und mit einer Steuerungseinrichtung (14) verbunden ist, **dadurch gekennzeichnet, dass** die Steuerungseinrichtung (14) eingerichtet ist, zumindest eine Motorspannung des Elektromotors (13) zum Erzeugen eines akustisch wahrnehmbaren Signals wiederholt umzupolen, ohne dass der Rotor (40) eine vollständige Drehung um die Drehachse (50) ausführt.

2. Orthopädietechnische Einrichtung nach Anspruch 1, wobei die Steuerungseinrichtung (14) eingerichtet ist, eine Umpolfrequenz zwischen 20Hz und 6000 Hz zu erzeugen.

3. Orthopädietechnische Einrichtung nach einem der Ansprüche 1 oder 2, wobei bei unterschiedlichen Umpolfrequenzen unterschiedliche Spannungsamplituden anliegen.

4. Orthopädietechnische Einrichtung nach einem der Ansprüche 1-3, wobei der Elektromotor (13) als bürstenloser Gleichstrommotor oder Kommutatormotor ausgebildet ist.

5. Verfahren zum Erzeugen eines akustischen Feedbacks einer orthopädietechnischen Einrichtung, nach Anspruch 1, mit zumindest einem Elektromotor (13), der einen Stator (30) und einen um eine Drehachse (50) verdrehbaren Rotor (40) aufweist und mit einer durch den Elektromotor (13) verstellbaren Komponente der orthopädietechnischen Einrichtung gekoppelt ist, **dadurch gekennzeichnet, dass** der Elektromotor (13) durch wiederholte Umpolung zumindest einer Motorspannung oszillierend betrieben wird, ohne dass der Rotor (40) eine vollständige Drehung um die Drehachse (50) ausführt.

6. Verfahren nach Anspruch 5, wobei als Motorspannung eine Rechteckspannung, Dreiecksspannung, Sägezahnspannung, Sinuskurvenspannung oder eine Mischform daraus verwendet wird.

7. Verfahren nach Anspruch 5 oder 6, wobei die Umpolung mit einer Frequenz zwischen 20Hz und 6000Hz betrieben wird.

8. Verfahren nach einem der Ansprüche 5-7, wobei ein Elektromotor (13) mit unterschiedlichen Umpolfrequenzen in zeitlicher Abfolge und/oder mehrere Elektromotore (13) mit unterschiedlichen Umpolfrequenzen gleichzeitig oder nacheinander betrieben werden.

9. Verfahren nach einem der Ansprüche 5-8, wobei die Amplitude der Motorspannung während der Umpolung verändert wird.

10. Verfahren nach einem der Ansprüche 5-9, wobei der Motor (13) als Kommutatormotor ausgebildet ist und die Motorspannung des Elektromotors (13) zum Erzeugen eines akustisch wahrnehmbaren Signals periodisch umgepolt wird.

11. Verfahren nach einem der Ansprüche 5-10, wobei der Elektromotor (13) als bürstenloser Gleichstrommotor ausgebildet ist und die Phasenlage des Drehfeldes des Elektromotors (13) zum Erzeugen eines akustisch wahrnehmbaren Signals wiederholt, insbesondere periodisch geändert wird.

12. Verfahren nach einem der Ansprüche 5-11, wobei eeeeder Rotor (40) zur Erzeugung eines akustisch wahrnehmbaren Signals um einen Drehwinkel von nicht mehr als 120°, insbesondere nicht mehr als 90° oszillierend bewegt wird.

## Claims

1. An orthopedic device comprising at least one electric motor (13), which has a stator and a rotor (40) which can rotate about an axis of rotation (50) and is coupled to a component (10, 20) of the orthopedic device which can be adjusted by the electric motor (13) and is connected to a control device (14), **characterized in that** the control device (14) is set up to repeatedly reverse the polarity of at least one motor voltage of the electric motor (13) to generate an acoustically perceptible signal, without the rotor (40) performing a complete rotation about the axis of rotation (50).

2. The orthopedic device as claimed in claim 1, wherein the control device (14) is set up to generate a polarity reversal frequency between 20 Hz and 6000 Hz.

3. The orthopedic device as claimed in either one of claims 1 and 2, wherein different voltage amplitudes are present at different polarity reversal frequencies.

4. The orthopedic device as claimed in any one of claims 1 to 3 wherein the electric motor (13) is designed as a brushless DC motor or commutator motor.

5. A method for generating acoustic feedback of an orthopedic device according to claim 1, comprising at least one electric motor (13), which has a stator (30) and a rotor (40) which can rotate about an axis of rotation (50) and is coupled to a component of the orthopedic device which can be adjusted by the electric motor (13), **characterized in that** the electric motor (13) is operated in an oscillating manner by repeated reversal of the polarity of at least one motor voltage, without the rotor (40) performing a complete rotation about the axis of rotation (50).

6. The method as claimed in claim 5, wherein a square-wave voltage, delta voltage, sawtooth voltage, sinusoidal curve voltage or a mixed form thereof is used as the motor voltage.

7. The method as claimed in claim 5 or 6, wherein the polarity reversal is operated at a frequency between 20 Hz and 6000 Hz.

8. The method as claimed in any one of the claims 5 to 7, wherein an electric motor (13) with different polarity reversal frequencies is operated in a time sequence and/or several electric motors (13) with different polarity reversal frequencies are operated simultaneously or successively.

9. The method as claimed in any one of the claims 5 to 8, wherein the amplitude of the motor voltage is changed during the polarity reversal.

10. The method as claimed in any one of the claims 5 to 9, wherein the motor (13) is designed as a commutator motor and the polarity of the motor voltage of the electric motor (13) is reversed periodically to generate an acoustically perceptible signal.

11. The method as claimed in any one of the claims 5 to 10, wherein the electric motor (13) is designed as a brushless DC motor and the phase of the rotating field of the electric motor (13) is repeated, in particular changed periodically, to generate an acoustically perceptible signal.

12. The method as claimed in any one of the claims 5 to 11, wherein the rotor (40) is moved in an oscillating manner by an angle of rotation of not more than 120°, in particular not more than 90°, to generate an acoustically perceptible signal.

## Revendications

1. Dispositif orthopédique comprenant au moins un moteur électrique (13) qui présente un stator et un rotor (40), pouvant tourner autour d'un axe de rotation (50), et qui est couplé à un composant (10, 20) du dispositif orthopédique, pouvant être déplacé par le moteur électrique (13), et qui est relié à un dispositif de commande (14),
**caractérisé en ce que** le dispositif de commande (14) est conçu pour inverser de manière répétée la polarité d'au moins une tension du moteur électrique (13), sans que le rotor (40) n'effectue un tour complet autour de l'axe de rotation (50), pour générer un signal acoustiquement perceptible.

2. Dispositif orthopédique selon la revendication 1,
dans lequel le dispositif de commande (14) est conçu pour générer une fréquence d'inversion de polarité comprise entre 20 Hz et 6000 Hz.

3. Dispositif orthopédique selon l'une des revendications 1 ou 2,
dans lequel des amplitudes de tension différentes sont appliquées pour des fréquences d'inversion de polarité différentes.

4. Dispositif orthopédique selon l'une des revendications 1 à 3,
dans lequel le moteur électrique (13) est conçu comme un moteur à courant continu sans balais.

5. Procédé pour générer un retour d'information acoustique d'un dispositif orthopédique selon la revendication 1, comprenant au moins un moteur électrique (13) qui présente un stator (30) et un rotor (40), pouvant tourner autour d'un axe de rotation (50), et qui est couplé à un composant du dispositif orthopédique, pouvant être déplacé par le moteur électrique (13),
**caractérisé en ce que** le moteur électrique (13) est exploité de manière oscillante par inversion répétée de la polarité d'au moins une tension du moteur, sans que le rotor (40) n'effectue un tour complet autour de l'axe de rotation (50).

6. Procédé selon la revendication 5,
dans lequel est prévu, comme tension du moteur, une tension rectangulaire, une tension triangulaire, une tension en dents de scie, une tension sinusoïdale ou une forme mixte de celles-ci.

7. Procédé selon la revendication 5 ou 6,
dans lequel l'inversion de la polarité est opérée à une fréquence comprise entre 20 Hz et 6000 Hz.

8. Procédé selon l'une des revendications 5 à 7,
dans lequel un moteur électrique (13) à différentes fréquences d'inversion de polarité est exploité en succession temporelle, et/ou plusieurs moteurs électriques (13) à différentes fréquences d'inversion de polarité sont exploités simultanément ou successivement.

9. Procédé selon l'une des revendications 5 à 8,
dans lequel l'amplitude de la tension du moteur est modifiée pendant l'inversion de la polarité.

10. Procédé selon l'une des revendications 5 à 9,
dans lequel le moteur (13) est un moteur à collecteur, et la polarité de la tension du moteur électrique (13) est périodiquement inversée pour générer un signal acoustiquement perceptible.

11. Procédé selon l'une des revendications 5 à 10,
dans lequel le moteur électrique (13) est réalisé sous la forme d'un moteur à courant continu sans balai, et la position de phase du champ magnétique rotatif du moteur électrique (13) est modifiée de manière répétée, en particulier périodiquement, pour générer un signal acoustiquement perceptible.

12. Procédé selon l'une des revendications 5 à 11,
dans lequel le rotor (40) est déplacé de manière oscillante d'un angle de rotation ne dépassant pas 120°, en particulier ne dépassant pas 90°, pour générer un signal acoustiquement perceptible.
